(19) Europäisches Patentamt
European Patent Office
Office européen des brevets

(11) Publication number: 0 080 284
A1

(12) # EUROPEAN PATENT APPLICATION

(21) Application number: **82305884.7**

(22) Date of filing: **05.11.82**

(51) Int. Cl.³: **C 07 D 498/04**, A 61 K 31/42
//
(C07D498/04, 263/00, 205/00),
(A61K31/42, 31/43)

(30) Priority: **25.11.81 GB 8135459**

(43) Date of publication of application: **01.06.83**
**Bulletin 83/22**

(84) Designated Contracting States: **CH DE FR GB IT LI NL**

(71) Applicant: **BEECHAM GROUP PLC, Beecham House Great West Road, Brentford Middlesex (GB)**

(72) Inventor: **Stirling, Irene, 38 Harrison Close, Reigate Surrey (GB)**
Inventor: **Bruton, Gordon, 61A Milton Road, Croydon Surrey (GB)**

(74) Representative: **Hesketh, Alan, Dr. et al, European Patent Attorney Beecham Pharmaceuticals Great Burgh Yew Tree Bottom Road, Epsom, Surrey, KT18 5XQ (GB)**

(54) **Derivatives of clavulanic acid, processes for their preparation and their use.**

(57) A compound of formula (II):

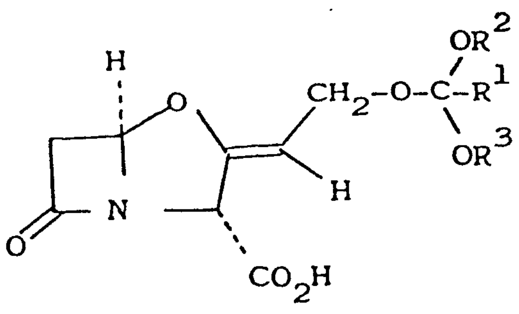

(II)

or salts or esters thereof wherein $R^1$ is hydrogen or a hydrocarbon group of 1 to 20 carbon atoms, and $R^2$ and $R^3$ are independently a hydrocarbon group of 1 to 20 carbon atoms, any of such hydrocarbon groups being optionally substituted.

EP 0 080 284 A1

0080284

TITLE MODIFIED
see front page

## CHEMICAL COMPOUNDS, PROCESSES FOR THEIR PREPARATION AND THEIR USE

This invention relates to derivatives of clavulanic acid and particular to a class of ether derivatives. These compounds have antibacterial and β-lactamase inhibtory qualities, and therefore are of use in the treatment of bacterial infection either alone or in a synergistic composition with other antibacterial agents, such as penicillins and cephalosporins.

British Patent Specification 1565209 claims clavulanic acid ethers of the formula (I):

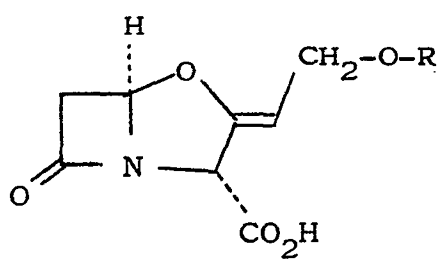

(I)

and salts and esters thereof wherein R is an inert etherifying organic group of up to 18 carbon atoms.

We have now discovered a novel class of compounds of the formula (II):

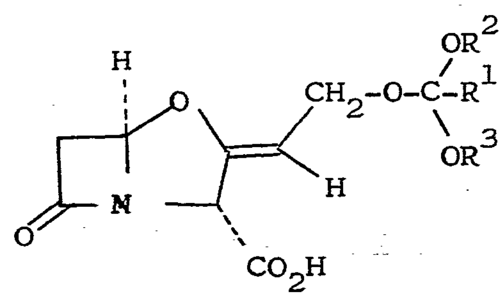

(II)

and salts and esters thereof wherein $R^1$ is hydrogen or a hydrocarbon group of 1 to 20 carbon atoms, and $R^2$ and $R^3$ are independently a hydrocarbon group of 1 to 20 carbon atoms, any of such hydrocarbon groups being optionally substituted.

Suitable substituents for $R^1$, $R^2$ and $R^3$ include $C_{1-6}$ alkanoylamino, halo, $C_{1-6}$ alkoxycarbonyl, $C_{1-6}$ alkanoyloxy, $C_{1-6}$ alkylthio, arylthio and esterified carboxy.

Suitable hydrocarbon groups for $R^1$, $R^2$ and $R^3$ include $C_{1-6}$ alkyl, aryl $(C_{1-6})$ alkyl and aryl. Preferably any aryl group is phenyl or substituted phenyl.

Most suitably $R^1$ is a hydrogen atom, methyl, ethyl, propyl, butyl, phenyl, benzyl or phenethyl group.

Preferably $R^1$ is hydrogen.

Most suitably $R^2$ is $C_{1-4}$ alkyl such as methyl, ethyl, isopropyl, n-propyl, n-butyl, isobutyl, t-butyl or sec-butyl, or phenyl or benzyl.

Preferably $R^2$ is methyl or ethyl.

Most suitably $R^3$ is $C_{1-4}$ alkyl such as methyl, ethyl, isopropyl, n-propyl, n-butyl, isobutyl, t-butyl or sec-butyl, or phenyl or benzyl.

Preferably $R^3$ is methyl or ethyl.

In one aspect it is convenient if $R^2$ and $R^3$ represent the same group, for example $R^2$ and $R^3$ are both methyl, or $R^2$ and $R^3$ are both ethyl.

Particular compounds of this invention include: benzyl 9-O-(dimethoxymethyl)clavulanate, lithium 9-O-(dimethoxymethyl)clavulanate, sodium 9-O-(dimethoxymethyl)-clavulanate, benzyl 9-O-(diethoxymethyl)clavulanate, lithium 9-O-(diethoxymethyl)clavulanate, sodium 9-O-(diethoxymethyl)clavulanate, benzyl 9-O-(1-phenyl-1,1-dimethoxymethyl)clavulanate, lithium 9-O-(1-phenyl-1,1-dimethoxymethyl)clavulanate, and sodium 9-O-(1-phenyl-1,1-dimethoxymethyl)clavulanate.

The major utility of the compounds of the formula (II) and salts and esters thereof is as pharmaceuticals and accordingly the salts and esters of the compounds of the formula (II) are preferably pharmaceutically acceptable. The compounds of this invention both pharmaceutically acceptable and non-pharmaceutically acceptable may be used as intermediates and also as antibacterial agents or β-lactamase inhibitors in non-pharmaceutical usage such as a disinfectant or paint additive.

Suitable pharmaceutically acceptable salts of the compounds of the formula (II) include metal salts such as aluminium, alkali metal salts such as sodium and potassium, and alkaline earth metal salts such as calcium or magnesium; and ammonium and substituted ammonium salts, for example those with lower alkylamines such as triethylamine, cycloalkylamines such as bicyclohexylamine, 1-ephenamine, N-ethylpiperidine and N-benzyl-β-phenethylamine.

Suitable esters of the compounds of the formulae (II) include those cleavable by biological methods such as enzy matic hydrolysis, <u>in-vivo</u> hydrolysis, and those cleavable by chemical methods such as hydrogenolysis, hydrolysis, electrolysis or photolysis.

Suitably the carboxylic acid is esterified by a group of the sub-formula (a), (b), (c), (d), (e) or (f):

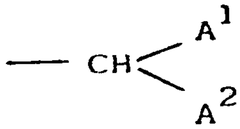 (a)

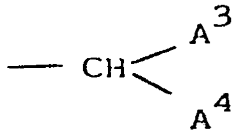 (b)

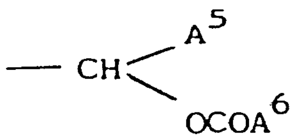 (c)

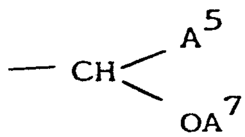 (d)

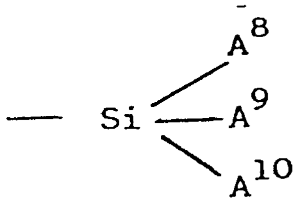 (e)

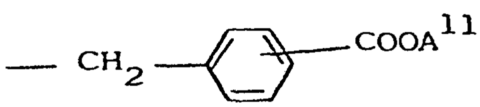 (f)

wherein $A^1$ is a hydrogen atom, $C_{1-6}$ alkanoyl or an $C_{1-5}$ alkyl group optionally substituted by $C_{1-7}$ alkoxy or $C_{1-7}$ carboxylic acyloxy, or an alkenyl or alkynyl group of up to 5 carbon atoms; $A^2$ is a hydrogen atom or a methyl group; $A^3$ is a phenyl group or a phenyl group substituted by a fluorine, chlorine or bromine atom or a nitro, $C_{1-3}$ alkyl or $C_{1-3}$ alkoxy group; $A^4$ is a hydrogen atom or a phenyl group or phenyl group substituted by a fluorine, chlorine or bromine atom or a nitro, $C_{1-3}$ alkyl or $C_{1-3}$ alkoxy group; $A^5$ is a hydrogen atom or a methyl group; $A^6$ is a $C_{1-4}$ alkyl, phenyl or $C_{1-4}$ alkoxy group or $A^5$ is

joined to $A^6$ to form a phthalidyl, dimethylphthalidyl or dimethoxyphthalidyl group; $A^7$ is a $C_{1-4}$ alkyl, phenyl, chlorophenyl or nitrophenyl group; $A^8$ is a $C_{1-4}$ alkyl or phenyl group; $A^9$ is a $C_{1-4}$ alkyl or phenyl group; $A^{10}$ is $C_{1-4}$ alkyl; and $A^{11}$ is $C_{1-4}$ alkyl: or $CHA^1A^2$ is a phenacyl or bromophenacyl group.

Favourably $A^1$ is a hydrogen atom or a methyl, ethyl, vinyl or ethenyl group. Favourably $A^2$ is a hydrogen atom. Favourably $A^3$ is a phenyl, p-bromophenyl, p-methoxyphenyl or p-nitrophenyl group. Favourably $A^4$ is a hydrogen atom. Favourably $A^6$ is a methyl, t-butyl or ethoxy group or is joined to $A^5$. Favourably $A^7$ is a methyl group.

Preferred groups of the sub-formula (a) include the methyl, ethyl and acetonyl groups.

Preferred groups of the sub-formula (b) include the benzyl and p-nitrobenzyl groups.

Preferred groups of the sub-formula (c) include the acetoxymethyl, pivaloyloxymethyl, α-ethoxycarbonyloxymethyl and phthalidyl groups.

BAD ORIGINAL

A preferred group of the sub-formula (d) is the methoxymethyl group.

Preferred groups of the sub-formula (e) include the trimethylsilyl, tert-butyldimethylsilyl and tert-butyldiphenylsilyl groups.

A preferred group of the sub-formula (f) is p-methoxycarbonylbenzyl.

Particularly preferred esterifying groups are the p-nitrobenzyl and phthalidyl groups.

Pharmaceutically acceptable in-vivo hydrolysable esters are those esters which hydrolyse in the human body to produce the parent acid or its salt. Such esters may be identified by administration to a test animal such as a rat or mouse by intravenous administration and thereafter examining the test animal's body fluids for the presence of the compound of the formula (II) or salt.

Suitable esters of this type include those of sub-formula (c) as hereinbefore defined.

BAD ORIGINAL

Compounds of this invention when in crystalline form may be solvated, for example hydrated.

The present invention provides a pharmaceutical composition which comprises a compound of the formula (II) or a pharmaceutically acceptable salt or pharmaceutically acceptable ester thereof and a pharmaceutically acceptable carrier. Preferably any such ester is in-vivo hydrolysable.

The compositions of the invention include those in a form adapted for oral, topical or parenteral use and may be used for the treatment of infection in animals for example mammals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives, disintegrant and the like in accordance with conventional pharmaceutical practice in the manner well understood by those skilled in the art of formulating antibiotics.

BAD ORIGINAL

Injectable or infusable compositions of a compound of the invention are particularly suitable as high blood levels of the compound can occur after administration by injection or infusion. Thus, one preferred composition aspect of this invention comprises a compound of the invention in sterile form and most suitably in sterile crystalline form.

The injectable solution of the compound of this invention may be made up in a sterile pyrogen-free liquid such as water, aqueous ethanol or the like.

An alternative approach to administering the compounds of this invention is to utilise an injectable suspension. Such suspensions may be made up in sterile water; sterile saline or the like, and may also contain suspending agents such as polyvinylpyrrolidone, lecithin or the like. Alternatively such compositions may be prepared in an acceptable oil suspending agent such as arachis oil or its equivalent. For use in such suspensions the compounds of this invention should be in the form of fine particles.

Unit dose compositions comprising a compound of this invention adapted for oral administration form a further suitable composition aspect of this invention.

Unit dose compositions comprising a compound of this invention adapted for topical administration are also presented by this invention. In this instance 'topical administration' also includes local administration to internal surfaces of mammary glands of cattle, for example during the treatment of mastitis by intra-mammary administration.

The compound of the invention may be present in the composition as sole therapeutic agent or it may be present together with other therapeutic agents such as a penicillin or cephalosporin. Considerable advantages accrue from the

inclusion of a penicillin or cephalosporin which shows instability to β-lactamases since the resulting composition shows enhanced effectiveness (synergy). Suitable penicillins, cephalosporins or other β-lactam antibiotics for inclusion in such synergistic compositions include not only those known to be highly susceptible to β-lactamases but also those which have a degree of intrinsic resistance to β-lactamases.

Suitable penicillins for inclusion in the compositions of this invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, sulbenicillin, piperacillin, and other known penicillins including pro-drugs therefor such as their in vivo hydrolysable esters such as the acetoxymethyl, pivaloyloxymethyl, α-ethoxycarbonyloxyethyl or phthalidyl esters of ampicillin, benzylpenicillin or amoxycillin, and aldehyde or ketone adducts of penicillins containing a 6-α-aminoacetamide side chain (such as hetacillin, metampicillin and analogous derivatives of amoxycillin) or α-esters of carbenicillin or ticarcillin such as their phenyl or indanyl α-esters.

A further group of suitable penicillins of interest for inclusion in the compositions of this invention include benzylpenicillin, phenoxymethylpenicillin, carbenicillin, azidocillin, propicillin, amoxycillin, epicillin, ticarcillin, cyclacillin, pirbenicillin, azlocillin, mezlocillin, sulbenicillin, piperacillin, and other known penicillins including pro-drugs therefor such as their in vivo hydrolysable esters such as the acetoxymethyl, pivaloyloxymethyl, α-ethoxycarbonyloxyethyl or phthalidyl esters of benzylpenicillin or amoxycillin, and α-esters of carbenicillin or ticarcillin such as their phenyl or indanyl α-esters.

Suitable cephalosporins for inclusion in the compositions of this invention include cefatrizine, cephaloridine, cephalothin, cefazolin, cephalexin, cephacetrile, cephamandole nafate, cephapirin, cephradine, 4-hydroxycephalexin, cefaparole, cephaloglycin, cefoperazone and other known cephalosporins or pro-drugs thereof.

Such compounds are frequently used in the form of a salt or hydrate of the like.

Naturally if the penicillin or cephalosporin present in the composition is not suitable for oral administration then the composition will be adapted for parenteral administration.

BAD ORIGINAL

Highly favoured penicillins for use in the compositions of this invention include ampicillin, amoxycillin, carbenicillin and ticarcillin. Such penicillins may be used as a pharmaceutically acceptable salt such as the sodium salt. Alternatively the ampicillin or amoxycillin may be used in the form of fine particles of the zwitterionic form (generally as ampicillin trihydrate or amoxycillin trihydrate) for use in an injectable suspension, for example in the manner hereinbefore described for a compound of this invention.

The preferred penicillin for use in the synergistic composition is amoxycillin, for example as its sodium salt or trihydrate.

Particularly suitable cephalosporins for use in the compositions of this invention include cephaloridine and cefazolin which may be in the form of a pharmaceutically acceptable salt for example the sodium salt.

When present together with a cephalosporin or penicillin, the ratio of a compound of the invention to the penicillin or cephalosporin agent may vary over a wide range of ratios, such as from 10:1 to 1:10 for example about 3:1, 2:1, 1:1, 1:2, 1:3, 1:4, 1:5 or 1:6, (wt/wt, based on pure free antibiotic equivalent).

Orally administrable compositions containing a compound of the invention will normally contain relatively more synergist than corresponding injectable compositions.

The total quantity of a compound of the invention in any unit dosage form will normally be between 25 and 1000 mg and will usually be between 50 and 500 mg, for example about 62.5, 100, 125, 150, 200 or 250 mg.

Compositions of this invention may be used for the treatment of infections of animals, for example mammals including humans, such infections being *inter alia* of the respiratory tract, the urinary tract and soft tissues in humans and mastitis in cattle.

Normally between 50 and 3000 mg of the compounds of the invention will be administered per day, for example at 1-6 doses, more usually as 2, 3 or 4 doses. However, for the treatment of more severe systemic infections or infections of particularly intransigent organisms higher doses may be used in accordance with clinical practice.

The penicillin or cephalosporin in the synergistic composition of this invention will normally be present at

BAD ORIGINAL

approximately the amount at which it is conventionally used which will usually be expected to be from about 62.5 to 3000 mg per dose, more usually about 125, 250, 500 or 1000 mg per dose.

One particularly favoured composition of this invention will contain from 150 to 1000 mg of amoxycillin as the trihydrate or sodium salt and from 25 to 500 mg of a compound of this invention.

A further particularly favoured composition of this invention will contain from 150 to 1000 mg of ampicillin or a pro-drug therefor and from 25 to 500 mg of a compound of this invention.

Most suitably this form of composition will contain ampicillin trihydrate, ampicillin anhydrate, sodium ampicillin, hetacillin, pivampicillin hydrochloride, bacampicillin hydrochloride, or talampicillin hydrochloride. Most suitably this form of the composition will contain a compound of this invention when in crystalline form.

Most suitably the preceding composition will contain from 200 to 700 mg of the penicillin component.

BAD ORIGINAL

Most suitably the preceding composition will comprise from 50 to 250 mg of a compound of this invention preferably in crystalline form.

Such compositions may be adapted for oral or parenteral use except when containing an in vivo hydrolysable ester of ampicillin or amoxycillin in which case the compositions will not be adapted for parenteral administration.

Another particularly favoured composition of this invention will contain from 200 to 2000 mg of carbenicillin, ticarcillin or a pro-drug therefor and from 50 to 500 mg of a compound of the invention.

Suitably this form of composition will contain di-sodium carbenicillin. Suitably this form of the composition will contain di-sodium ticarcillin.

More suitably this form of the composition will contain from 75 to 250 mg of a compound of this invention preferably in crystalline form. Such compositions containing di-salts of carbenicillin and ticarcillin will be adapted for parenteral administration.

BAD ORIGINAL

The present invention also provides a method of treating bacterial infection in humans or domestic mammals which comprises the administration of a composition of this invention.

Commonly the infection treated will be due to a strain of Staphylococcus aureus, Klebsiella aerogenes, Escherichia coli, Proteus sp., Bacteroides fragilis or the like. The organisms believed to be most readily treated by an antibacterially effective amount of a compound of this invention is Staphylococcus aureus. The other organisms named are more readily treated by using a synergistically effective amount of the compound of the invention and a penicillin or cephalosporin. The administration of the two components may take place separately but in general we prefer to use a composition containing both the synergist and the penicillin or cephalosporin.

BAD ORIGINAL

The present invention also provides a process for the preparation of a compound of the formula (II) or salt or ester thereof which process comprises the reaction of a compound of the formula (III):

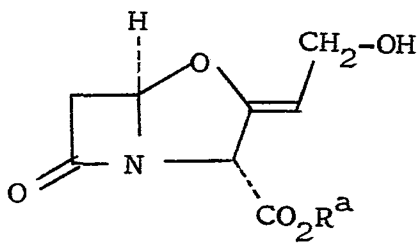

(III)

or reactive derivative thereof, wherein $R^a$ is a carboxy-blocking group; with a compound of the formula (IV):

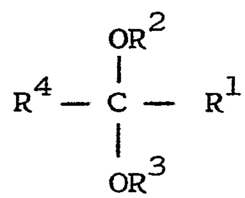

(IV)

wherein $R^1$, $R^2$ and $R^3$ are as defined in relation to a compound of the formula (II), and $R^4$ is a displaceable group; and thereafter if necessary:

i) removing any carboxy-blocking group $R^a$,

ii) converting the product into a free acid salt or ester.

Suitably $R^4$ is a displaceable group, such as a $C_{1-20}$ hydrocarbon oxy group, for example an aryloxy, aryl $(C_{1-6})$ alkoxy or $C_{1-6}$ alkoxy group.

Preferably $R^4$ is $C_{1-6}$ alkoxy such as methoxy or ethoxy.

It is believed that this reaction is an equilibrium reaction and therefore it is preferred that $R^4$ be chosen such that $R^4H$ is removed or consumed during the reaction, such removal for example would be conveniently performed by evaporation, or under reduced pressure, if $R^4$-H is volatile.

The reaction is suitably performed in an inert organic solvent such as dimethylformamide or halogenated hydrocarbons for example dichloromethane or chloroform. If the nature of the compound of the formula (IV) permits it may be more convenient to perform the reaction in the absence of solvent, the compound of the formula (IV) effectively being the solvent.

The reaction may be conveniently be performed at an ambient or elevated temperature, for example between 15°C and 80°C, more suitably between 15°C and 60°C, and most suitably at about 30° to 40°C.

In another aspect the reaction may be performed under a partial vacuum such as 10 to 20 mm Hg, which serves, where appropriate, to remove the by-product $R^4$-H from the reaction mixture. If this partial vacuum removes reagents or solvent from the reaction mixture then of course these may be replenished.

In a preferred embodiment an acid catalyst is present, for example a Lewis acid or protonic acid. Suitably the catalyst is an organic acid such as acetic acid.

Suitable carboxyl-blocking derivatives for the group $-CO_2R^a$ in formulae (III) include salts, esters, and anhydride derivatives of the carboxylic acid. The

derivative is one which may readily be cleaved at a later stage of the reaction. The salts need not be pharmaceutically acceptable. Suitable salts include inorganic salts, for example metal salts such as silver or mercuric salt, or alkali metal salts such as the lithium or sodium salt, and tertiary amine salts.

Suitable ester-forming carboxyl-blocking groups are those which may be removed under conventional conditions. Such groups for $R^a$ include benzyl, p-methoxybenzyl, 2,4,6-trimethylbenzyl, 3,5-di-t-butyl-4-hydroxybenzyl, benzoylmethyl, p-nitrobenzyl, 4-pyridylmethyl, 2,2,2-trichloroethyl, 2,2,2-tribromoethyl, acetonyl, diphenylmethyl, triphenylmethyl, 2-benzyloxyphenyl, 4-methylthiophenyl, pentachlorophenyl, p-toluenesulphonylethyl, methoxymethyl, a silyl, stannyl or phosphorus-containing group, an oxime radical of formula $-N{=}CHR^o$ where $R^o$ is aryl or heterocyclic, or an in-vivo hydrolysable ester.

The carboxyl group may be regenerated from any of the above esters by usual methods appropriate to the particular $R^a$ group, for example, base - catalysed hydrolysis, or by enzymically - catalysed hydrolysis, or by hydrogenation. The hydrolysis must of course be carried out under conditions to which the groups on the rest of the molecule are stable.

When it is desired to produce a compound of formula (II) in the form of a free acid or salt by the

process of this invention, a compound of formula (III) is generally employed wherein $R^a$ is a carboxyl-blocking group. For the preparation of a compound of formula (II) in the form of a pharmaceutically acceptable ester, it is convenient to employ a compound of formula (III) wherein $R^a$ represents the desired ester group.

Preferably the process of this invention is performed on a compound of the formula (III) wherein $R^a$ is an ester-forming group.

The compounds of the formula (III) are disclosed in British Patent Specifications 1508977 and 1508978.

The following Examples serve to illustrate the invention.

Example 1

Benzyl 9-O-(dimethoxymethyl) clavulanate

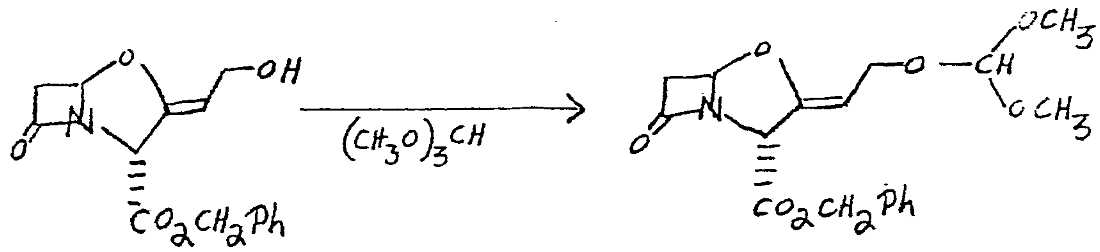


Benzyl clavulanate (2.89g; 10 mmole) was dissolved in trimethylorthoformate (15ml) and warmed to 35°C while being placed under vacuum (14 mm Hg). After 4 hours the reaction mixture was evaporated to an oil which was chromatographed on silica gel (toluene/ethyl acetate 4:1 as eluent) to give the title ester in 90% yield, 3.27g.

I.R. (film) 1800, 1749 and 1693 cm $^{-1}$.

N.M.R. ($CDCl_3$) δ 3.01(1H, d, J 17Hz, 6β-CH), 3.32(6H, s, $OCH_3$ x 2), 3.52(1H, dd, J 17 and 3 Hz, 6α-CH), 4.17(2H, d, J 8Hz, 9-$CH_2$), 4.88(1H, m, 8-CH), 5.08(2H, m, 3-CH and $(MeO)_2$ CH), 5.2(2H, s, $CH_2Ph$), 5.7(1H, d, J 3Hz, 5-CH) and 7.39(5H, s, aromatics).

Example 2

Lithium 9-O-(dimethoxymethyl)clavulanate

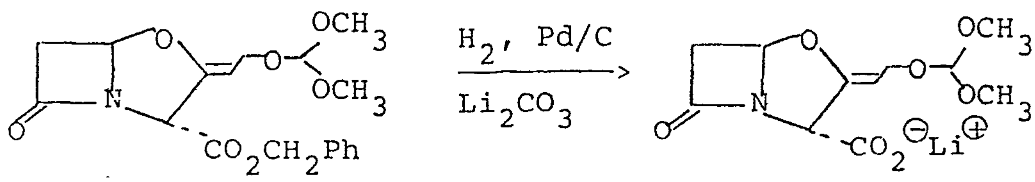


Benzyl 9-O-(dimethoxymethyl)clavulanate (3.3336 g, 9.18 mmol) in tetrahydrofuran (10 ml) was added to prehydrogenated catalyst 10% Pd/C (0.8 g) in aqueous tetrahydrofuran (20 ml) containing lithium carbonate (0.3393 g, 4.59 mmol). The mixture was hydrogenated for 20 minutes by which time the starting material had been consumed. The catalyst was filtered off and washed with water; the filtrate and aqueous washings were evaporated and the residue triturated with acetone. The solid thus formed was purified by chromatography on silica gel (ethyl acetate : isopropanol : water, 5 : 3 : 2 as eluent) affording Lithium 9-O-(dimethoxymethyl) clavulanate.

I.R. (Nujol) 1783, 1771, 1703 and 1624 $cm^{-1}$.

N.M.R. ($CD_3COCD_3$) $\delta$ 2.90 (1H, d, *J*17Hz, 6β-CH), 3.19 (6H, s, 2 x $OCH_3$), 3.46 (1H, dd, *J*17 and 3Hz, 6α-CH), 4.0 and 4.07 (2H, d, ABq, *J*11 and 7Hz, 9-$CH_2$), 4.51 (1H, br.s, 3-CH), 4.64 (1H, dt, *J*7 and 1Hz, 8-CH), 5.02 (1H, s, O-C<u>H</u>$(OMe)_2$), and 5.60 (1H, d, *J*3Hz, 5-CH).

BAD ORIGINAL

Example 3

Benzyl 9-0-(diethoxymethyl)clavulanate

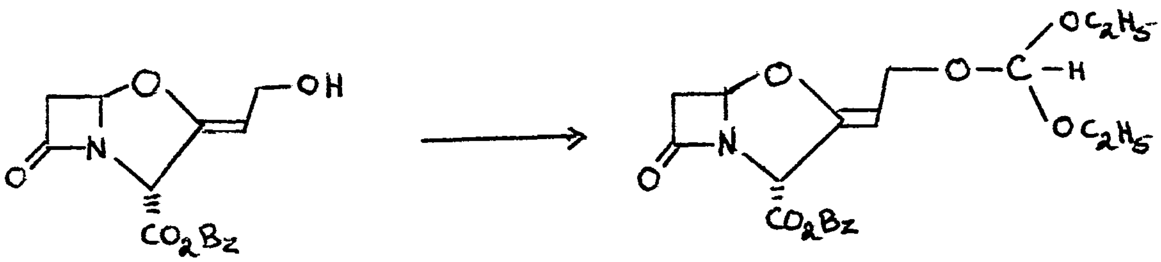

Benzyl clavulanate (1.0 g; 3.5 m.mol.) was dissolved in triethylorthoformate (15 ml), in a long neck, round bottom flask. The reaction vessel was evacuated using a water pump, to remove methanol formed during reaction and heated to 50°C in a water bath. The reaction mixture was maintained under vacuum and at 50°C for 25 min, after which thin layer chromatography indicated that all of the starting material had been consumed. The excess triethylorthoformate was removed under reduced pressure to yield a pale yellow oil. The oil was subjected to column chromatography eluting with ethyl acetate: cyclohexane (1:3). The fractions containing the desired material (Rf = 0.42, ethylacetate:cyclohexane, 1:2) were combined and the solvent removed under reduced pressure to give a colourless oil (0.8 g, 71%).

$\nu_{max}$ ($CHCl_3$) 1800, 1750, and 1700 $cm^{-1}$.

δ ($CDCl_3$) 1.20 (6H, t, J 7HZ, $OCH_2C\underline{H}_3$), 3.00 (1H, d, J 17Hz, 6β-C$\underline{H}$), 3.48 (1H, dd, J 3 and 17 Hz, 6α-C$\underline{H}$), 3.55 (4H, q, J 7Hz, $OC\underline{H}_2CH_3$), 4.18 (2H, d, J 6Hz, 9-$CH_2$), 4.85 (1H, t, 8-C$\underline{H}$), 5.08 and 5.14 (each 1H, s, 3-C$\underline{H}$ and $\underline{H}$-C($OEt)_2$), 5.16 (2H, s, $CH_2Ph$), 5.65 (1H, d, J 3Hz, 5-C$\underline{H}$), 7.35 (5H, s, $CH_2Ph$).

BAD ORIGINAL

Example 4

Lithium 9-O (diethoxymethyl) clavulanate

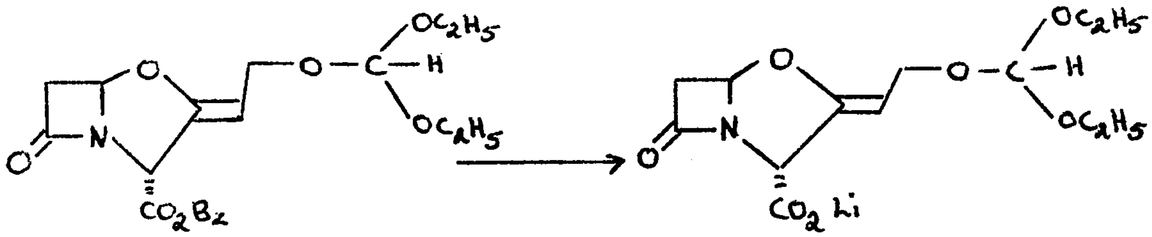

Benzyl 9-O - (diethoxymethyl) clavulanate (0.8g; 2m mol.) and lithium carbonate (0.15g) in 10% aqueous tetrahydrafuran (10ml), was hydrogenated for 15min. at room temperature using 10% palladium on carbon (0.27g) as catalyst. Thin layer chromatography indicated that all of the starting ester had been hydrogenated. The reaction mixture was then filtered and the catalyst washed with 10% aqueous tetrahydrofuran (10ml). The combined filtrates were concentrated to yield a thick syrup, which gave a white crystalline solid on addition of acetone. The solid was filtered off and recrystallised from aqueous acetone to give the desired salt (0.45g; 68%).

$\nu_{max.}$ (KBr) 1770, 1690 and 1620 $cm^{-1}$

δ(DMSO) 1.20 (6H, t, J 7Hz, $OCH_2\underline{CH_3}$), 2.85 (1H, d, J 17Hz, 6β-CH), 3.3 - 3.6 (5H, m, 6α-C$\underline{H}$ and $\overline{OCH_2}CH_3$), 4.02 (2H, d, J 7Hz, 9-$\underline{CH_2}$), 4.53 (1H, s, 3-$\underline{CH}$), 4.65 (1H, t, J 7Hz, 8-$\underline{CH}$), 5.10 (1H, s, $\underline{Hc}$(OFt)$_2$), 5.59 (1H, d, J 4Hz, 5-C$\underline{H}$).

ORIGINAL

Example 5

p-Nitrobenzyl 9-O-(α,α-dimethoxybenzyl)clavualante

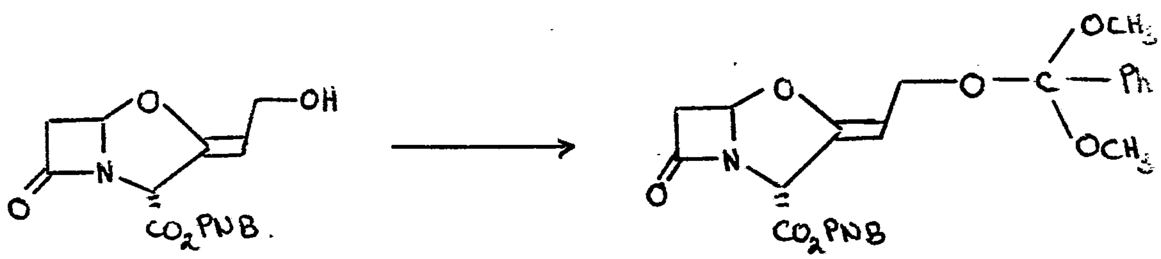

To p-nitrobenzyl clavulanate (1.25 g, 3.8 m. mol) was added trimethylorthobenzoate (30 ml) and the reaction vessel was warmed in a water bath at 45°C and swirled until the clavulanate had dissolved. When all of the clavulanate was in solution acetic acid (0.5 ml) was added and the vessel immediately evacuated to water-pump pressure. The reaction vessel was maintained under vacuum at 45°C with occasional stirreing until thin layer chromatography indicated that all of the clavulanate had reacted (approx. 1½ hour). The reaction mixture was then poured into ethyl acetate (50 ml). The organic layer was washed with saturated sodium bicarbonate solution (2 x 60 ml) brine (1 x 50 ml) and dried over anhydrous magnesium sulphate. Removal of the ethyl acetate under reduced pressure yielded a mobile oil from which the excess trimethylorthobenzoate was removed by vacuum distillation (0.03 mm Hg, 40°C). The resulting yellow oil was subjected to silica column chromatography eluting with ethyl acetate: cyclohexane (2:3). The fractions containing the desired ester (Rf = 0.3, ethyl acetate:cyclohexane, 2:3) were combined and the solvent removed in vacuo to yield the title compound as a colourless oil (1.1g; 60%).

$\nu_{max}$ ($CHCl_3$) 1800, 1750, and 1700 $cm^{-1}$.

δ ($CDCl_3$), 2.96 (1H, d, J 17Hz, 6β-CH), 3.12 (6H, s, $OCH_3$), 3.44 (1H, dd, J 4 and 17 Hz, 6α-CH), 3.92 (2H, d, J 7 Hz, 9-$CH_2$), 4.80 (1H, t, J 7Hz, 8-CH), 5.05 (1H, brS, 3-CH), 5.28 (2H, s, $CH_2Ar$), 5.60 (1H, d, J 4Hz, 5-CH), 7.25-7.60 and 8.24 (7H, m, ArH)

BAD ORIGINAL

DEMONSTRATION OF EFFECTIVENESS

In a standard MIC test the compound of Example 2 was tested as a synergist in combination with amoxycillin:

| | Staph Aureus Russell | Klebsiella Aerogenes E70 | E.coli JT39 |
|---|---|---|---|
| Amoxycillin alone | 500 | 500 | 2000 |
| Amoxycillin + the compound of Ex. 2 at 1.0 μg/ml | 1.25 | 6.2 | 31 |
| Amoxycillin + the compound of Ex. 2 at 5.0 μg/ml | 0.6 | 1.6 | 4.0 |
| The compound of Ex. 2 alone | 62 | 62 | 31 |

Claims

1. A compound of formula (II):

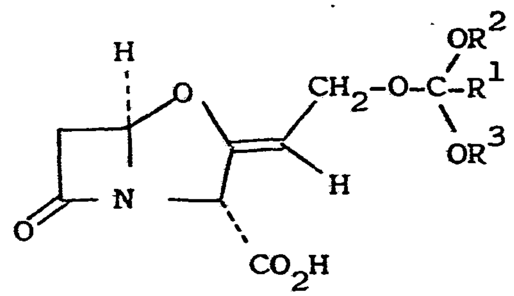

(II)

or salts or esters thereof wherein $R^1$ is hydrogen or a hydrocarbon group of 1 to 20 carbon atoms, and $R^2$ and $R^3$ are independently a hydrocarbon group of 1 to 20 carbon atoms, any of such hydrocarbon groups being optionally substituted.

2. A compound as claimed in claim 1 wherein optional substituents for $R^1$, $R^2$ and $R^3$ are selected from $C_{1-6}$ alkanoylamino, halo, $C_{1-6}$ alkoxycarbonyl, $C_{1-6}$ alkanoyloxy, $C_{1-6}$ alkylthio, arylthio and esterified carboxy.

3. A compound as claimed in claim 1 or claim 2 wherein $R^1$, $R^2$ and $R^3$ are the same or different and each is $C_{1-6}$ alkyl, aryl $(C_{1-6})$ alkyl or aryl.

4. A compound as claimed in claim 1 or claim 2 wherein $R^1$ is hydrogen.

5. A compound as claimed in any one of claims 1 to 4 wherein $R^2$ and $R^3$ represent the same group and are methyl or ethyl.

6. A compound as claimed in claim 1 selected from the following:

benzyl 9-O-(dimethoxymethyl)clavulantate, lithium 9-O-

(dimethoxymethyl)clavulanate, sodium 9-O-(dimethoxymethyl)-clavulanate, benzyl 9-O-(diethoxymethyl)clavulanate, lithium 9-O-(diethoxymethyl)clavulanate, sodium 9-O-(diethoxymethyl)clavulanate, benzyl 9-O-(1-phenyl-1,1-dimethoxymethyl)clavulanate, lithium 9-O-(1-phenyl-1,1-dimethoxymethyl)clavulanate, and sodium 9-O-(1-phenyl-1,1-dimethoxymethyl)clavulanate.

7. A pharmaceutcial composition comprising a compound as claimed in claim 1 or a pharmaceutically acceptable salt or pharmaceutically acceptable ester thereof and a pharmaceutically acceptable carrier.

8. A pharmaceutical composition as claimed in claim 7 which further comprises a penicillin or cephalosporin.

9. A method of treating bacterial infection in humans or domestic mammals which comprises the administration of a pharmaceutical composition as claimed in claim 7 or claim 8.

10. A process for the preparation of a compound as claimed in claim 1 or a salt or ester thereof which process comprises the reaction of a compound of the formula (III):

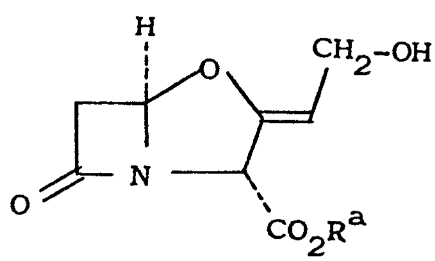

(III)

or reactive derivative thereof, wherein $R^a$ is a carboxy-blocking group; with a compound of the formula (IV):

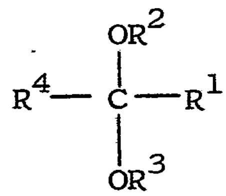 (IV)

wherein $R^1$, $R^2$ and $R^3$ are as defined in relation to a compound of the formula (II), and $R^4$ is a displaceable group; and thereafter if necessary:

i) removing any carboxy-blocking group $R^a$,

ii) converting the product into a free acid salt or ester.

0080284

European Patent Office

# PARTIAL EUROPEAN SEARCH REPORT

which under Rule 45 of the European Patent Convention shall be considered, for the purposes of subsequent proceedings, as the European search report

Application number

EP 82 30 5884

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | GB - A - 1 595 176 (BEECHAM)<br>* claims * | 1,7,8,10 | C 07 D 498/04<br>A 61 K 31/42//<br>(C 07 D 498/04<br>263/00<br>205/00)<br>(A 61 K 31/42<br>31/43) |
| | -- | | |
| A | GB - A - 1 572 259 (BEECHAM)<br>* claims | 1,7,8,10 | |
| | ----- | | |
| | | | TECHNICAL FIELDS SEARCHED (Int. Cl. 3) |
| | | | C 07 D 498/00<br>A 61 K 31/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-8,10

Claims searched incompletely: 9

Claims not searched:

Reason for the limitation of the search:

Method for treatment of the human or animal body by surgery or therapy (see article 52(4) of the European Patent Convention).

| Place of search | Date of completion of the search | Examiner |
|---|---|---|
| The Hague | 14-02-1983 | CHOULY |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document